## Europäisches Patentamt
## European Patent Office
## Office européen des brevets

(19)

(11) Publication number: **0 028 962**
**B1**

(12)

# EUROPEAN PATENT SPECIFICATION

(45) Date of publication of patent specification: **11.04.84**

(51) Int. Cl.³: **A 61 F 5/44,** A 61 F 1/00

(21) Application number: **80401528.7**

(22) Date of filing: **28.10.80**

(54) Apparatus for the continence of digestive stomas and anal incontinence.

(30) Priority: **30.10.79 ES 485537**
**20.05.80 ES 491637**
**20.05.80 ES 491638**

(43) Date of publication of application:
**20.05.81 Bulletin 81/20**

(45) Publication of the grant of the patent:
**11.04.84 Bulletin 84/15**

(84) Designated Contracting States:
**AT BE CH DE FR GB IT LI LU NL SE**

(56) References cited:
**DD - A - 131 619**
**DE - A - 1 541 262**
**DE - A - 2 806 405**
**FR - A - 2 041 444**
**FR - A - 2 251 302**
**FR - A - 2 373 272**
**US - A - 2 455 859**
**US - A - 2 699 781**
**US - A - 3 744 063**
**US - A - 3 750 194**
**US - A - 3 863 622**

(73) Proprietor: **Voltas Baro, Juan**
**Mtrio de Urdax No 15 Atico**
**Pamplona (Navarra) (ES)**

(73) Proprietor: **Ortiz Hurtado, Hector**
**Paseo Sarasate No 7 Sexto Izqda.**
**Pamplona (Navarra) (ES)**

(72) Inventor: **Voltas Baro, Juan**
**Mtrio de Urdax No 15 Atico**
**Pamplona (Navarra) (ES)**
Inventor: **Ortiz Hurtado, Hector**
**Paseo Sarasate No 7 Sexto Izqda.**
**Pamplona (Navarra) (ES)**

(74) Representative: **Mongrédien, André et al,**
**c/o BREVATOME 25, rue de Ponthieu**
**F-75008 Paris (FR)**

Courier Press, Leamington Spa, England.

Apparatus for the continence of digestive stomas and anal incontinence

The present invention relates to an apparatus for the continence of digestive stomas, and anal incontinence; those digestive stomas can be both abdominal and perineal.

In order to obtain continence of the digestive stomas, an apparatus known under the name of "Maclet (r)" is at present used.

This apparatus consists of two parts: a magnetic ring and a magnetic plug. The ring is placed around the stoma and the plug within it. This system gives satisfactory results only in colostomies, but not in ileostomies.

In any event, the said apparatus can today not be applied to the perineal region.

Other systems for treatment of incontinence can be cited as illustrative of the known art, especially the following patents: DD—A—131619, FR—A—2041444 and US—A—3863622.

The object of the present invention is to provide an apparatus which can be applied in any location of the stomas and in any type of stoma, and which also serves for anal incontinences of any degree.

The apparatus of the invention is characterized by the fact that it comprises a sphincter formed of a flexible band which has at least two longitudinal rows of staggered chambers. These chambers have flexible hermetically sealed walls. In addition, the chambers are interconnected with each other. One of the chambers bears connected to it a conduit through which, by means of a suction-impeller pump of subcutaneous location, a fluid is introduced and extracted, which makes it possible to fill and empty the different chambers or bubbles. When the chambers are filled, they are inflated to their maximum capacity, while when they are evacuated, they are deflated, the two walls of each chamber being practically against each other.

The strip is intended to be placed around the intestine near the stoma, on the outside thereof.

The strip being arranged in the manner indicated, the plunger of the valve is actuated, the pump aspirates the content of the chambers, evacuating them, whereby the decompression of the sphincter is obtained. On the other hand, when the pump is compressed, the fluid is again forced into the chambers filling them and the chambers are inflated, acquiring a given pressure, compressing and closing the digestive tube, thus avoiding the discharge of the solid, liquid or gaseous elements thereof.

The apparatus of the invention can be provided with an electronic device intended to advise the patient that the pressure within the digestive tube has reached given values which make it advisable to decompress the sphincter.

The sphincter of the invention may be constructed in different models and dimensions depending on the requirements and use thereof. For example, the strip may be of a height of 1 to 1 1/2 cm with a length which varies depending on the ostomy of the patient, for instance between 6 and 12 cm, and 2 mm in thickness. The strip will consist of two rows of three chambers, staggered with each other, of a height of 0,5 to 0,75 cm, which are arranged close together. The length of the chambers may vary between 2 and 4 cm. The different chambers communicate with each other, discharging through a tube which extends from one of the chambers, which tube extends up to the chamber of the manually actuated suction-impeller pump.

Of the two walls which define each chamber, the wall which will be directed towards the wall of the intestine is of greater flexibility than the outer wall, so that upon the inflation or deflation the chambers experience only a change in the position of the inner wall.

The inner wall of the chambers, in addition to being flexible, can be elastic.

The sphincter with its chambers as well as the outlet tube will preferably consist of silicone or some other material of similar properties which is tolerated by the body. Nevertheless, on one of the sides there may be placed a plate of different material which permits better anchoring in the tissue and stimulates fibrosis, such as teflon, dacron (Trade Marks) etc.

In order that the nature of the apparatus of the invention may be more easily understood, a more detailed description thereof is given below with reference to the accompanying drawings in which a few possible embodiments are shown, given by way of illustration and not of limitation.

In the drawings, identical parts or parts of identical function have been designated by the same reference numbers.

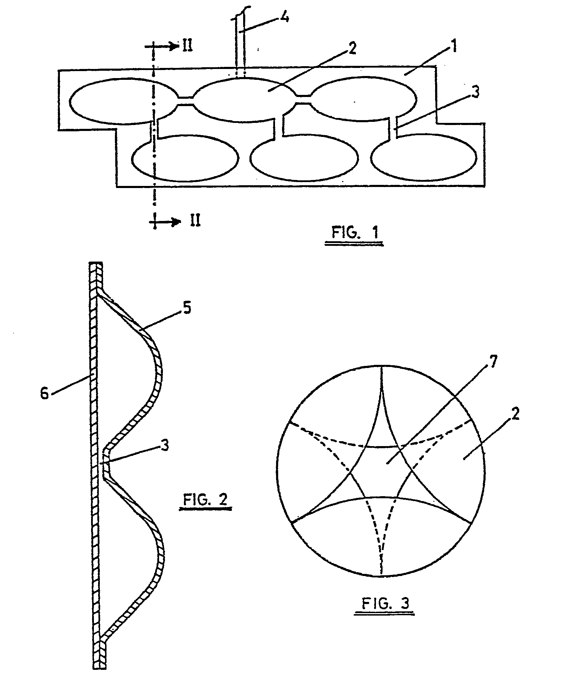

Fig. 2 is an elevation of the band or strip which constitutes the sphincter;

Fig. 2 is a cross section on a larger scale, along the line II—II of Fig. 1;

Fig. 3 is a plan view, on a larger scale, of the shaped sphincter;

As can be noted from Fig. 1, the sphincter is formed from a strip 1 of suitable material such as silicone, on which there have been shaped a number of chambers 2 with hermetically sealed walls, which communicate with each other through small passageways 3, so that the pressure which prevails in all the chambers is practically the same. From one of these chambers a conduit 4 extends to a manually operated suction-impeller pump, which may be of any nature.

This pump will operate in such a manner that when the valve is depressed the fluid contained in the chambers 2 is aspirated, emptying them, whereby the two walls of said chambers come against each other. On the other hand, when the pump is actuated, the fluid will be forced into the chambers 2, which will be inflated.

As shown in Fig. 2, the chambers have one wall 5 of greater flexibility than the other component wall 6 of the strip 1. The wall 5 of greater flexibility will be the wall which faces the intestine when placed around the latter.

In the example described, the strip 1 comprises two rows of chambers 2 aligned in each row, those of one row being staggered with respect to those of the other. In Fig. 2 there is shown the intercommunication passage 3 between two chambers which is obtained between the walls 5 and 6 which define said chambers.

The wall 5 will be of great flexibility and may furthermore be elastic in nature.

The wall 6 may be covered on one of its faces by a material which facilitates its anchoring to the tissue, which material may consist of teflon, dacron (Trade Marks), etc.

The strip of Fig. 1 is arranged in the manner shown in Fig. 3, winding it from the intestine near the stoma and connecting the ends of the strip. In this way, the chambers 2 when they are inflated will reduce to a minimum the central space 7 occupied by the intestine, which will be subjected to pressure, preventing the discharge of solid, liquid and even gaseous elements. When the pump is operated, the chambers 2 are deflated, whereby the stoma is freed, permitting the discharge of the elements contained in the intestine.

As already indicated, there may be placed in the intestine near the stoma and in an intramural location, an electronic device which advises the patient when a given pressure at which decompression of the sphincter is advisable has been reached.

Having sufficiently described the nature of the invention as well as the manner of reducing it to practice, it should be pointed out that the arrangements indicated above are capable of modifications in detail insofar as they do not change its fundamental principle.

## Claims

1. An apparatus for the continence of digestive stomas and anal incontinence of the type comprising an artifical sphincter intended to be placed around the intestine, on the outside of it, near the stoma to be fed through a conduit by means of a pump, the said sphincter being formed of a flexible strip (1) having a hermetically sealed chamber the inner wall of which is of greater flexibility than the outer wall characterized by the fact that the strip (1) is provided with a series of chambers (2) arranged in at least two longitudinal rows with the chambers staggered and interconnected.

2. An apparatus according to claim, characterized by the fact that the inner wall of the chamber is elastic.

## Revendications

1. Dispositif pour la fermeture de stomies intestinales et pour l'incontinence anale, du type comportant un sphincter artificiel destiné à être placé autour de l'intestin et sur sa face externe, à proximité des stomies, et à être alimenté par l'intermédiaire d'une conduit au moyen d'une pompe, ledit sphincter étant formé par une bande flexible (1) comportant une chambre obturée hermétiquement, dont la paroi interne est d'une flexibilité plus grande que sa paroi externe, caractérisé par le fait que la bande (1) est munie d'une série de chambres (2) agencées selon au moins deux rangées longitudinales, ces chambres étant en quinconce et en communication mutuelle.

2. Dispositif selon la revendication 1, caractérisé par le fait que la paroi interne de la chambre est élastique.

## Patentansprüche

1. Vorrichtung zum Verschluß von künstlichen Darmausgängen und bei analer Inkontinenz der Ausführung, die einen künstlichen Schließmuskel aufweist, der zum Anlegen um den Darm herum an dessen Außenseite nahe dem künstlichen Darmausgang und zur Versorgung durch eine Leitung mittels einer Pumpe vorgesehen ist, wobei der genannte Schließmuskel von einem flexiblen Streifen (1) mit einer hermetisch abgedichteten Kammer gebildet ist, deren Innenwand eine größere Flexibilität als die Außenwand aufweist, gekennzeichnet, durch die Tatsache, daß der Streifen (1) mit einer Reihe von Kammern (2) versehen ist, die in wenigstens zwei Längsreihen angeordnet sind, wobei die Kammern gestaffelt und untereinander verbunden sind.

2. Vorrichtung nach Anspruch 1, gekennzeichnet durch die Tatsache, daß die Innenwand der Kammer elastisch ist.

0 028 962

FIG. 1

FIG. 2

FIG. 3